# FASCICULE DE BREVET EUROPEEN

(11) **EP 0 668 071 B1**
(45) Date de publication et mention de la délivrance du brevet: **04.09.1996**
(21) Numéro de dépôt: 95400174.9
(22) Date de dépôt: 26.01.1995
(51) Int. Cl.: A61K 7/42

(54) **Compositions cosmétiques antisolaires stables, fluides et/ou fluidifiables**
Lagerstabile, fluide und/oder fluidisierbare kosmetische Sonnenschutzmittel
Stable, fluid and /or fluidizable cosmetic sunscreening composition

(30) Priorité: 18.02.1994 FR 9401861
(43) Date de publication de la demande: 23.08.1995
(73) Titulaire: L'OREAL, F-75008 Paris (FR)
(72) Inventeur: Allard, Delphine, F-92700 Colombes (FR); Ascione, Jean-Marc, F-75018 Paris (FR)
(74) Mandataire: Andral, Christophe André Louis

(56) Documents cités:
- EP-A- 0 433 086
- EP-A- 0 518 773

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), fluides et/ou fluidifiables, stables, un de leur procédé de préparation, ainsi que leur utilisation dans l'application cosmétique susmentionnée. Plus précisément encore, elle concerne des compositions antisolaires à propriétés améliorées se présentant sous la forme d'émulsions de type huile-dans-eau (support cosmétiquement acceptable) et comprenant, à titre d'agents photoprotecteurs agissant par blocage physique du rayonnement (réflecteurs et/ou diffuseurs d'UV), des nanopigments minéraux à base d'oxydes métalliques, et en particulier d'oxyde de titane, et à titre d'agents stabilisants des silicates mixtes de métaux alcalins et/ou alcalino-terreux. Elle concerne par ailleurs un procédé de stabilisation d'émulsions de type huile-dans-eau contenant des nanopigments.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo-toxiques ou photo-allergiques. Il est donc souhaitable de filtrer aussi le rayonnement UV-A.

De nombreuses compositions destinées à la photoprotection (UV-A et/ou UV-B) de la peau ont été proposées à ce jour et, dans ce domaine, on observe que l'utilisation de nanopigments minéraux (c'est à dire de pigments dont la taille moyenne des particules primaires n'excède généralement pas 100 nm) à base d'oxydes métalliques, et en particulier d'oxyde de titane, devient de plus en plus fréquente, compte tenu du fait que ces dernières substances, lorsqu'elles sont associées avec des filtres UV classiques (principalement des composés organiques capables d'absorber les rayonnements nocifs) permettent d'obtenir des indices de protection très élevés.

Pour diverses raisons liées en particulier à un meilleur confort d'utilisation (douceur, émollience et autres), les compositions antisolaires actuelles se présentent le plus souvent sous la forme d'une émulsion de type huile-dans-eau (c'est à dire un support constitué d'une phase continue dispersante aqueuse et d'une phase discontinue dispersée huileuse) dans laquelle on a introduit, à des concentrations diverses, les nanopigments susmentionnés, le cas échéant en association avec d'autres filtres UV classiques, et qui peuvent être présents tant dans la phase aqueuse de l'émulsion que dans sa phase huileuse (encore appelée phase grasse). Dans ces émulsions classiques, qui contiennent en outre des agents émulsionnants (ou tensio-actifs) et d'éventuels additifs cosmétiques usuels tels que parfums, colorants ou conservateurs, la taille des globules constituant la phase grasse est généralement supérieure à plusieurs microns.

L'un des inconvénients des compositions antisolaires connues à ce jour et appartenant au type ci-dessus (émulsion H/E contenant des nanopigments), et plus particulièrement de celles contenant des nanopigments d'oxyde de titane TiO₂, est que, une fois appliquées sur la peau sous la forme d'un film, elles engendrent sur cette dernière un effet de blanchissement qui est cosmétiquement indésirable et généralement peu apprécié des utilisateurs. Cet effet est d'autant plus marqué que la concentration en nanopigments dans l'émulsion est élevée. Pour éviter ce problème, il serait bien entendu possible de mettre en oeuvre des quantités réduites de nanopigments, mais les émulsions résultantes, qui conduiraient certes à des films présentant une transparence acceptable sur la peau, n'offriraient alors plus une protection convenable dans le domaine des UV, ce qui limite fortement l'intérêt d'une telle opération.

Une autre difficulté réside par ailleurs dans le fait que les émulsions classiques antisolaires à base de nanopigments protecteurs conduisent, après application sur la peau, à une distribution irrégulière, non homogène, voire grossière, desdits nanopigments sur cette peau, ce qui peut nuire à la qualité de l'effet de photoprotection global recherché. Cette mauvaise répartition des nanopigments que l'on constate à la surface de la peau est souvent liée au fait qu'il existe, au niveau de l'émulsion initiale même (avant application), un manque substantiel d'homogénéité (mauvaise dispersion du pigment dans son support).

Enfin, on observe, pour certaines des émulsions antisolaires ci-dessus, et bien que ces dernières contiennent des agents émulsionnants (ou tensio-actifs) comme indiqué précédemment, un certain manque de stabilité dans le temps, ce qui nuit à leur conservation une fois conditionnées. Ce manque de stabilité se traduit, dans les faits, par des phénomènes plus ou moins prononcés de décantation des nanopigments au sein de l'émulsion, voire de séparation (déphasage) entre les phases aqueuse et huileuse de cette émulsion.

La présente Demanderesse a déja proposé, dans la demande de brevet français déposée sous le numéro 94/01455 (FR-A-2 715 843), des émulsions antisolaires améliorées de type H/E et contenant des nanopigments minéraux à base d'oxydes métalliques, qui présentent à la fois une excellente transparence sur la peau, une très bonne efficacité de protection contre les UV, une bonne stabilité et une parfaite homogénéité tant avant qu'après application sur la peau (c'est à dire que les nanopigments sont très bien dispersés dans l'émulsion initiale d'une part et sur la peau après application d'autre part). Plus précisémment encore, il a été montré dans la demande précitée qu'il était possible de remédier aux différents inconvénients liés à l'emploi et à la présence de nanopigments photoprotecteurs dans les émulsions H/E classiques de l'art antérieur, en mettant en oeuvre des émulsions H/E spécifiques dites "ultrafines", et pour lesquelles la taille moyenne des globules constituant la phase grasse est comprise dans des limites bien déterminées, à savoir entre 100 et 1000 nm, et de préférence entre 100 et 500 nm, lesdites émulsions ultrafines de type H/E étant elles-mêmes de préférence obtenues selon une technique d'émulsification par inversion de phase. Les formulations antisolaires décrites dans la demande FR 94/01455 (FR-A-2 715 843) répondent typiquement à la composition suivante : (i) phase aqueuse : de 50 à 95 % en poids, de préférence de 70 à 90 % en poids, par rapport à l'ensemble de la formulation ; (ii) phase huileuse : de 5 à 50 % en poids, de préférence de 10 à 30 % en poids, par rapport à l'ensemble de la formulation ; (iii) nanopigments : de 0,5 à 40 % en poids, de préférence de 1 à 30 % en poids, par rapport à l'ensemble de la formulation ; (iv) (co)émulsionnant(s) : de 0,5 à 20 % en poids, de préférence de 2 à 10% en poids, par rapport à l'ensemble de la formulation. Les formulations ci-dessus sont de préférence obtenues selon un procédé de préparation comprennant les étapes essentielles suivantes : (a) on mélange sous agitation, en présence d'un système émulsionnant convenablement sélectionné (agents émulsionnants de type non ioniques et choisis, seuls ou en mélanges, parmi les alcools gras polyoxyéthylénés et/ou polyoxypropylénés, et les esters d'acides gras et de polyols, éventuellement polyoxyéthylénés et/ou polyoxypropylénés, le sytème émulsionnant retenu présentant de préférence un HLB global compris entre 9,5 et 11,5 environ, et encore plus préférentiellement proche de 10), une phase grasse d'une part et une phase aqueuse d'autre part, ledit mélange se faisant à une température supérieure à la température d'inversion de phase (TIP) du milieu, de manière à obtenir une émulsion de type eau-dans-huile, (b) on ramène la température de l'émulsion ainsi obtenue à une température inférieure à ladite température d'inversion de phase, ce par quoi l'on obtient une émulsion ultrafine de type huile-dans-eau, (c) on procède à l'introduction des nanopigments minéraux lors de la mise en oeuvre de l'étape (a) et/ou à l'issue de l'étape (b). Toutes choses étant égales par ailleurs (i.e. à composition chimique et concentrations identiques), on a pu ainsi démontrer dans la demande française susmentionnée qu'une composition antisolaire conforme à l'invention s'y rapportant, par le simple ajustement de la taille des globules huileux à une valeur convenable telle qu'indiquée ci-avant, présentait systématiquement, au niveau notamment de sa tranparence sur la peau, de sa stabilité, de son homogénéïté et de son pouvoir de protection, des propriétés améliorées par rapport à une même composition antisolaire ne satisfaisant pas au critère susmentionné de taille des globules d'huile.

Or, poursuivant ses travaux dans le domaine, la Demanderesse a été par la suite amenée à constater que les compositions décites dans la demande FR 94/01455 (FR-A-2 715 843) présentaient elles-mêmes un inconvénient, à savoir que lorsque l'on cherche à préparer des compositions telles que ci-dessus qui soient en outre fluides, et ceci notamment dans le but de disposer de produits qui soient facilement vaporisables, alors on observe que les compositions obtenues manquent, à plus ou moins long terme, d'une certaine stabilité, ce manque de stabilité se traduisant dans les faits par l'apparition d'un phénomène de décantation progressive du nanopigment au sein de l'émulsion.

La présente invention a justement pour but de résoudre le problème ci-dessus.

Ainsi, à la suite d'importantes recherches menées sur la question, il a maintenant été trouvé par la Demanderesse qu'il est possible de remédier aux problèmes de manque de stabilité attachés aux émulsions ultrafines (100 nm<Φ_{globules}<1000 nm) à base de nanopigments, simplement en introduisant dans ces dernières des silicates mixtes de métaux alcalins et/ou alcalino-terreux. L'introduction de ces silicates mixtes dans l'émulsion ultrafine provoque certes un certain épaississement de la composition (qui varie selon les quantités introduites), mais les silicates mixtes étant des agents de viscosité thixotropes, les compositions résultantes peuvent, en fait, être par la suite facilement fluidifiées soit par action mécanique, par exemple par agitation du produit juste avant emploi, ou lors de son application même (frottement) sur la peau ou encore par cisaillement de ce dernier au moment de son passage à travers le corps de pompe d'un vaporisateur, soit par dilution à l'eau. Par ailleurs, cette introduction ne perturbe en rien l'ensemble des propriétés avantageuses qui sont attachées de manière inhérente aux émulsions ultrafines H/E à base de nanopigments, et se trouvent ainsi notamment conservées la bonne transparence sur la peau, la bonne efficacité de protection UV, I'homogénéité tant avant qu'après application sur la peau, et enfin l'absence de déphasage entre les phases grasse et aqueuse. L'effet de stabilisation général (absence de décantation d'une part et de déphasage d'autre part) engendré par la présence des silicates mixtes dans les émulsions H/E à base de nanopigments est d'autant plus inattendu et surprenant que ces derniers, lorsqu'ils sont introduits dans une émulsion ultrafine exempte de nanopigments, provoquent chez celle-ci un phénomène rapide de séparation entre les phases aqueuse et huileuse de l'émulsion (émulsion non stable).

Toutes ces découvertes sont à la base de la présente invention.

Ainsi, conformément à l'un des objets de la présente invention, il est maintenant proposé de nouvelles compositions cosmétiques, en particulier antisolaires, fluides et/ou fluidifiables, comprenant, dans un support cosmétiquement acceptable de type émulsion huile-dans-eau dans lequel la taille moyenne des globules constituant la phase huileuse est comprise entre 100 nm et 1000 nm, des nanopigments minéraux à base d'oxydes métalliques à titre d'agents photoprotecteurs, et qui sont caractérisées par le fait qu'elles contiennent en outre au moins un silicate mixte de métaux alcalins et/ou alcalino-terreux.

L'invention a également pour objet l'utilisation d'un silicate mixte de métaux alcalins et/ou alcalino-terreux pour la stabilisation d'une émulsion ultrafine de type H/E contenant des nanopigments, ainsi que le procédé de stabilisation correspondant qui consiste à introduire dans une émulsion ultrafine de type H/E contenant des nanopigments, soit lors de la préparation même de ladite émulsion, soit après ladite préparation (émulsion déja réalisée), au moins un silicate mixte de métaux alcalins et/ou alcalino-terreux.

Un autre objet de l'invention est constitué par un procédé particulier de préparation des compositions selon l'invention.

D'autres caractéristiques, aspects et avantages de l'invention apparaitront à la lecture de la description détaillée qui va suivre.

Selon la présente invention, on entend par silicates mixtes d'alcalins et/ou d'alcalino-terreux des silicates d'origine naturelle ou synthétique dont la composition présente, à côté des atomes de silicium et d'oxygène (motifs silicates), des cations métalliques (assurant la neutralité chimique de l'ensemble) choisis parmi soit au moins deux métaux alcalins différents, soit au moins deux métaux alcalino-terreux différents, soit au moins un métal alcalin et au moins un métal alcalino-terreux. De tels silicates présentent une structure chimique analogue à celle de l'hectorite (argile naturelle). De préférence, on fait appel à des silicates mixtes d'origine synthétique, car ces produits sont en effet exempts ou substantiellement exempts d'impuretés, notamment de silice libre. Les silicates mixtes sont par ailleurs connus pour leurs propriétés épaississantes et thixotropes.

De préférence, on utilise des silicates mixtes contenant au moins un métal alcalino-terreux.

Encore plus préférentiellement, on utilise des silicates mixtes contenant au moins un métal alcalino-terreux en association avec au moins un métal alcalin.

Les métaux alcalins sont de préférence choisis parmi le litium, le sodium et le potassium.

Les métaux alcalino-terreux sont, quant à eux, de préférence choisis parmi le magnésium et le calcium.

Selon un mode particulièrement préféré de réalisation de la présente invention, on utilise au moins un silicate mixte de magnésium, de lithium et de sodium.

Ces produits sont bien connus de l'homme de l'art. Ils peuvent être en particulier synthétisés selon le procédé enseigné dans le brevet US 3 586 478. Certains sont par ailleurs des produits commerciaux, et sont notamment vendus sous les noms de marque LAPONITE® (LAPONITES DS, D, XLS ou XLG entre autres) par la Société LAPORTE Industries, Ltd.

Les oxydes métalliques (nanopigments) utilisables dans le cadre de la présente invention sont tous ceux déja connus en soi pour leur activité photoprotectrice. Ainsi, ils peuvent être notamment choisis parmi les oxydes de titane, de zinc, de fer, de zirconium, de cérium ou leurs mélanges.

De tels nanopigments d'oxydes métalliques, enrobés ou non enrobés, sont des produits déja bien connus de l'homme de l'art et sont en particulier décrits dans la demande de brevet EP-A- 0 518 773. A titre de nanopigments commerciaux complémentaires non cités dans la demande précitée mais également utilisables dans le cadre de la présente invention, on peut en outre mentionner les produits vendus sous les noms de marque UVT M 160, UVT M 212 et UVT M 262 par la Société KEMIRA, et MT 100 SAS par la Société TAYCA.

Selon un mode préféré de réalisation des compositions antisolaires selon l'invention, on fait appel à des nanopigments minéraux à base d'oxyde de titane, qui offrent en effet la meilleure efficacité au niveau photoprotection. Par ailleurs, on notera que l'effet cosmétique indésirable de blanchissement de la peau évoqué dans la partie introductive de la présente description est particulièrement marqué avec ce type de nanopigments. Cet oxyde de titane peut se présenter sous une forme cristallisée de type rutile et/ou anatase, et/ou sous une forme amorphe ou substantiellement amorphe. Comme indiqué précédemment, ce pigment peut être alors enrobé ou non enrobé, mais de préférence on utilise des pigments enrobés, par exemple par de l'alumine et/ou du stéarate d'aluminium et/ou de la silice.

Selon leur caractère lipophile, ou au contraire hydrophile, plus ou moins prononcé, les nanopigments pourront être présents soit dans la phase grasse de l'émulsion, soit dans la phase aqueuse, ou bien même encore dans les deux phases à la fois.

La taille moyenne des particules primaires des nanopigments présents dans les compositions selon l'invention est généralement comprise entre 5 nm et 100 nm, de préférence entre 10 et 50 nm.

Bien entendu, les compositions antisolaires conformes à l'invention peuvent en outre contenir un ou plusieurs filtres solaires organiques classiques (absorbeurs), actifs dans l'UV-A et/ou l'UV-B, hydrophiles ou lipophiles. A titre d'exemples, ces filtres complémentaires peuvent être choisis parmi l'acide 2-phényl benzimidazole 5-sulfonique et ses sels, les dérivés cinnamiques tels que par exemple le p-méthoxycinnamate de 2-éthylhexyle, les dérivés salicyliques comme par exemple le salicylate de 2-éthylhexyle et le salicylate d'homomenthyle, les dérivés du camphre comme par exemple le 3(4-méthylbenzylidène)camphre ou l'acide camphosulfonique (1,4 divinylbenzène), les dérivés de triazine tels que la 2,4,6-tris [p-(2'-éthylhexyl-1'-oxycarbonyl)anilino] 1,3,5-triazine, les dérivés de la benzophénone tels que la 2-hydroxy 4-méthoxybenzophénone, les dérivés du dibenzoylméthane tels que le 4-tert-butyl 4'-méthoxydibenzoylméthane, les dérivés de β,β-diphénylacrylate tels que le α-cyano-β,β-diphénylacrylate de 2-éthylhexyle, les dérivés de l'acide p-aminobenzoïque comme par exemple le paradiméthylaminobenzoate d'octyle, l'anthranilate de menthyle, les polymères filtres et silicones filtres décrits dans la demande WO-93-04665. D'autres exemples de filtres organiques sont donnés dans la demande de brevet EP-A 0 487 404.

La nature de la phase grasse rentrant dans la composition des émulsions selon l'invention n'est pas critique et elle peut ainsi être constituée par tous les composés qui sont déja connus de façon générale comme convenant pour la fabrication d'émulsions de type huile dans eau. En particulier, ces composés peuvent être choisis, seuls ou en mélanges, parmi les différents corps gras, les huiles d'origine végétale, animale ou minérale, les cires naturelles ou synthétiques, et analogues.

Parmi les huiles pouvant rentrer dans la composition de la phase grasse, on peut notamment citer :
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre ce karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, le myristate de butyle, le myristate d'isopropyle, le myristate de cétyle, le palmitate d'isopropyle, I'adipate d'isopropyle, l'adipate d'éthylhéxyle, le stéarate de butyle, le stéarate d'héxadécyle, le stéarate d'isopropyle, le stéarate d'octyle, le stéarate d'isocétyle, I'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-α-oléfines.

Comme autres huiles utilisables dans les émulsions selon l'invention, on peut encore citer les benzoates d'alcools gras en C₁₂-C₁₅ (Finsolv TN de FINETEX), les alcools gras tels que l'alcool laurique, cétylique, myristique, stéarilique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en C₁₀-C₁₈, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques, notamment ceux qui sont déja utilisés de manière habituelle dans la fabrication et l'obtention des compositions cosmétiques antisolaires.

Selon une caractéristique essentielle des compositions conformes à la présente invention, la taille moyenne des particules liquides (ou globules) de phase grasse au sein de la phase aqueuse dispersante doit être comprise dans des limites bien particulières, à savoir entre 100 nm et 1000 nm. De préférence, cette taille moyenne est comprise entre 100 nm et 500 nm. Encore plus préférentiellement, la distribution en taille des globules huileux est telle que la plupart desdits globules (i.e au moins 90% en nombre) présentent une taille comprise entre les bornes indiquées ci-avant.

De manière classique, la phase aqueuse dispersante peut être constituée par de l'eau, ou un mélange d'eau et d'alcool(s) polyhydrique(s) comme par exemple glycérol, propylèneglycol et sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvants cosmétiques classiques hydrosolubles.

Parmi les adjuvants cosmétiques classiques susceptibles d'être contenus dans la phase aqueuse et/ou dans la phase grasse des émulsions conformes à l'invention (selon leur caractère hydro- et/ou liposoluble), on peut citer notamment les épaississants ioniques ou non ioniques, les adoucissants, les antioxydants, les opacifiants, les stabilisants, les émollients, les agents répulsifs contre les insectes, les agents hydratants, les vitamines, les parfums, les conservateurs, les charges, les séquestrants, les colorants, ou tout autre ingrédient habituellement utilisé dans le domaine des produits solaires.

Les émulsions conformes à l'invention contiennent en outre généralement des tensio-actifs ou émulsionnants particuliers dont l'emploi a été rendu nécessaire pour la préparation et l'obtention de l'émulsion ultrafine. Ce point sera détaillé par la suite. Elles peuvent en outre contenir des co-émulsionnants spécifiques dont le rôle est, lors de la préparation de l'émulsion, de diminuer de manière substantielle la quantité d'agents tensio-actifs nécessaire à la réalisation de l'émulsion.

A titre indicatif, les formulations antisolaires conformes à l'invention présentent généralement les compositions suivantes :
(i) phase aqueuse : de 50 à 95 % en poids, de préférence de 70 à 90 % en poids, par rapport à l'ensemble de la formulation,
(ii) phase huileuse : de 5 à 50 % en poids, de préférence de 10 à 30 % en poids, par rapport à l'ensemble de la formulation,
(iii) nanopigments : de 0,5 à 40 % en poids, de préférence de 1 à 30 % en poids, par rapport à l'ensemble de la formulation,
(iv) silicate(s) mixte(s) : de 0,05 à 5 % en poids, de préférence de 0,1 à 3,5% en poids, par rapport à l'ensemble de la formulation,
(iv) (co)émulsionnant(s) : de 0,5 à 20 % en poids, de préférence de 2 à 10% en poids, par rapport à l'ensemble de la formulation.

Elles présentent en outre une viscosité de préférence inférieure 15 000 cps, encore plus préférentiellement inférieure à 5 000 cps (mesurée sur viscosimètre Brookfield RVT modèle DV2, à 5 tr/mn et avec disque n°5)

Les compositions stables conformes à l'invention peuvent être préparées par tout procédé convenable connu en soi et se ramenant essentiellement à l'introduction, dans une émulsion ultrafine H/E contenant des nanopigments, de silicates mixtes de métaux alcalins et/ou alcalino-terreux. Elles peuvent ainsi être obtenues soit en introduisant le ou les silicates mixtes dans une émulsion ultrafine préalablement réalisée (première variante), laquelle pourra elle-même avoir été obtenue par tout moyen connu en soi (ultra-sons, mélangeurs/homogénéiseurs hautes pressions, inversion de phases ou autres), soit en intégrant cette étape d'introduction de silicates au niveau même d'au moins une des étapes d'un procédé classique de préparation d'une émulsion ultrafine (deuxième variante) ; dans cette dernière variante, les émulsions obtenues ne se distinguent de celles qui seraient obtenues par le même procédé mais sans mettre en oeuvre de silicates, que par la seule présence desdits silicates au sein desdites émulsions. Il en découle que pour obtenir des émulsions conformes à l'invention présentant des caractéristiques de composition chimique (hors silicates) et de structure désirées, il suffit de mettre en oeuvre un procédé classique et connu de préparation d'émulsions ultrafines H/E contenant des nanopigments dont on sait qu'il conduit aux émulsions avec les caractéristiques voulues, mais dans lequel on aura en outre intégré une étape d'introduction de silicates. Cette règle de correspondance entre les émulsions ne contenant pas de silicates et celles en contenant s'applique bien entendu également, par analogie, à la première variante évoquée ci-avant.

Nonobstant ce qui précède, un procédé particulièrement préféré de préparation des compositions selon l'invention va maintenant être développé.

Ce procédé repose sur la technique de fabrication des émulsions H/E par inversion de phase. Cette technique est, dans son principe, bien connue de l'homme de l'art et est notamment décrite dans l'article "Phase Inversion Emusification", par Th Förster et al, paru dans Cosmetics & Toiletries, vol. 106, Decembre 1991, pp 49-52. Son principe est ainsi le suivant : on prépare une émulsion (introduction de l'eau dans l'huile) à une température qui doit être supérieure à la température d'inversion de phase (ou TIP) du système, c'est à dire la température à laquelle l'équilibre entre les propriétés hydrophiles et lipophiles du ou des émulsionnants mis en oeuvre est atteint ; à température élevée (>TIP), I'émulsion est de type eau-dans-huile, et au cours de son refroidissement, à la température d'inversion de phase, cette émulsion s'inverse pour devenir une émulsion de type cette fois huile-dans-eau, et ceci en étant passée auparavant par un état de microémulsion.

Selon l'invention, des nanopigments doivent être présents dans l'émulsion ultrafine H/E finale. Ainsi, selon un premier mode possible de mise en oeuvre du procédé de préparation selon l'invention, l'inversion de phase de l'émulsion est conduite en présence des nanopigments photoprotecteurs décrits précédemment; selon un deuxième mode de mise en oeuvre de ce procédé, ces nanopigments ne sont introduits qu'après que l'émulsion par inversion de phase ait été obtenue. Il est bien entendu possible de cumuler les deux modes de mise en oeuvre.

Conformément aux première et seconde variantes indiquées ci-dessus, les silicates mixtes peuvent, quant à eux, être introduits dans le milieu lors de l'étape même de mise en oeuvre de l'inversion de phase (première variante), soit, de préférence, à l'issue de cette étape (deuxième variante). Là encore, le cumul des deux variantes est possible.

L'une des difficultés pour la mise en oeuvre d'un procédé tel que ci-dessus réside dans le choix convenable du système émulsionnant qui doit être approprié au résultat recherché.

Les systèmes émulsionnants qui doivent ainsi être retenus dans le cadre de l'invention sont ceux qui permettent effectivement d'obtenir des émulsions ultrafines par inversion de phase (100 nm<Φ_{globules}<1000 nm), stables, et dans lesquelles les nanopigments se trouvent dispersés de façon fine et homogène.

Les travaux de la Demanderesse ont montré que, à cet effet, les systèmes émulsionnants convenant à la présente invention devaient être des émulsionnants de type non ioniques et, plus particulièrement encore, être choisis parmi les alcools gras polyoxyéthylénés et/ou polyoxypropylénés (i.e des composés obtenus par réaction entre un alcool gras aliphatique, comme l'alcool béhénique ou l'alcool cétylique, avec de l'oxyde d'éthylène ou de l'oxyde de propylène ou un mélange oxyde d'éthylène/oxyde de propylène) et les esters d'acides gras et de polyols, éventuellement polyoxyéthylénés et/ou polyoxypropylénés (i.e des composés obtenus par réaction d'un acide gras, comme l'acide stéarique ou l'acide oléique, avec un polyol, comme par exemple un alkylèneglycol ou du glycérol ou un polyglycérol, éventuellement en présence d'oxyde d'éthylène ou d'oxyde de propylène ou d'un mélange oxyde d'éthylène/oxyde de propylène), ou leurs mélanges. Par ailleurs, et de préférence, le système émulsionnant retenu présentera un HLB global (comme cela est bien connu, on désigne par HLB (Hydrophilic-Lipophilic Balance, au sens de Griffin ; voir J. Soc. Cosm. Chem. 1954 (vol 5), pp 249-256) I'équilibre entre le caractère hydrophile et le caractère lipophile de l'agent tensioactif) compris entre 9,5 et 11,5 environ, avantageusement proche de 10, de manière à permettre l'obtention d'une inversion de phase à une température inférieure à 90°C (TIP<90°C).

De façon inattendue et surprenante, la présence des nanopigments et/ou des silicates minéraux dans le système initial à émulsionner ne perturbe en rien les mécanismes qui sont naturellement mis en jeu dans un procédé d'émulsification par inversion de phase; au contraire, on aboutit à une émulsion ultrafine dans laquelle les particules constituant les nanopigments et les silicates mixtes sont elles-mêmes maintenues à l'état d'une fine dispersion (absence d'agglomération, ou taille d'agglomérats extrèmement faible), parfaitement homogène et stable dans le temps.

Les détails d'un procédé de préparation conforme à l'invention apparaitront dans les exemples donnés ci-après.

Un troisième objet de la présente invention est constitué par l'utilisation des compositions selon l'invention telles que ci-dessus définies comme, ou pour la fabrication de, compositions protectrices de l'épiderme humain ou des cheveux contre les rayons ultraviolets, ou comme compositions antisolaires. Les compositions peuvent alors être conditionnées sous la forme de crèmes, de laits, de gels crèmes, ou bien encore de lotions fluides, en particulier de lotions fluides vaporisables, les compositions selon l'invention présentant en effet la propriété avantageuse d'être aisément diluables à l'eau tout en restant stables.

Le procédé de traitement cosmétique de la peau ou des cheveux destiné à les protéger contre les effets des rayons UV, en particulier de ceux contenus dans le rayonnement solaire, consiste à appliquer sur ceux-ci une quantité efficace d'une composition cosmétique telle que définie ci-dessus.

Des exemples concrets, illustrant l'invention, vont maintenant être donnés.

### EXEMPLES

On a préparé (par la technique d'inversion de phase), puis comparé, différentes émulsions ultrafines (Φ_{globules}<1 µm) contenant ou non des nanopigments de TiO₂ et/ou des silicates mixtes (Formulations F1 à F7).

Dans le cas des nanopigments, les matières premières utilisées étaient les suivantes :
- oxyde de titane TiO2 de qualité nanopigmentaire, vendu sous la référence MT 100 T par la Société TAYCA
- ou oxyde de titane TiO2 de qualité nanopigmentaire, vendu sous la référence TIOVEIL AQ par la Société TIOXIDE.

Les silicates mixtes utilisés étaient des silicates mixtes de magnésium, de lithium et de sodium, vendus sous le nom de marque de LAPONITE XLG par la Société LAPORTE Industries.

Les compositions chimiques (% en poids ramenés à l'ensemble de la formulation) de ces formulations étaient les suivantes (voir aussi Tableau I ci-après) :

| *Phase A* : | |
|---|---|
| - Alcool cétylstéarylique à 12 moles d'oxyde d'éthylène (EUMULGIN B1 de HENKEL) | 3,3 % |
| - Stéarate de glycérol (TEGIN 90 de GOLDSCHMIDT) | 1,7 % |
| - Polydécène hydrogéné (ETHYL FLO 362 NF de ETHYL CORP) | 10 % |
| - Dioctylcyclohexane (Cetiol S de HENKEL) | 6 % |
| - Cyclométhicone | 4 % |

| *Phase B* : | |
|---|---|
| - oxyde de titane TiO2 | de 0 % à 5 % |

| *Phase C* : | |
|---|---|
| - silicates mixtes | de 0% à 3,5 % |

| *Phase D* : | |
|---|---|
| - Glycérine | 3 % |
| - Eau | qsp 100 % |

| *Phase E* : | |
|---|---|
| - Conservateurs | qs |

Le mode opératoire qui a été suivi pour préparer ces formulations était le suivant : les phases grasse (*A*) et aqueuse (*D*) ont été toutes deux préalablement portées à une température de l'ordre de 90°C ; lorsque la phase (*B*) contient le pigment référencé MT 100 T, cette dernière a été introduite et dispersée dans la phase grasse (*A*), et ceci sous agitation énergique au moyen d'une turbine type MORITZ (1000 t/mn) ; puis on a ajouté la phase aqueuse (*D*) dans la dispersion résultante, toujours sous agitation mécanique, cette étape d'émulsification étant conduite à 80°C, c'est à dire à une température supérieure à la température d'inversion de phase du système ; après avoir observé l'inversion de phase en ramenant la température du milieu vers 40 °C, on introduit dans l'émulsion ultrafine résultante la phase (*B*) lorsque celle-ci contient le pigment référencé TIOVEIL AQ, puis la phase (*C*) qui aura été préalablement dispersée dans de l'eau à 60 °C, et enfin la phase (*E*).

Pour chacune des formulations ainsi obtenues, on a ensuite évalué leur stabilité en conservation, et ceci à température ambiante (TA) d'une part et à 45°C d'autre part, en observant l'apparition ou non (i) d'un déphasage entre les phases aqueuse et huileuse de l'émulsion et/ou (ii) d'une décantation du pigment au sein de l'émulsion.

Les résultats sont rassemblés dans le tableau I donné ci-après. Dans ce tableau, l'expression "OK" signifie qu'aucun déphasage et qu'aucune décantation n'ont été observées après 2 semaines de conservation.

Ces résultats démontrent clairement la supériorité des formulations F5 à F7 conformes à l'invention au niveau de leur stabilité.

**TABLEAU I**

| **FORMULATION n°** | **COMPOSITION** | | **STABILITE** | |
|---|---|---|---|---|
| | Nanopigments | Silicates | à TA | à 45 °C |
| **F1** (comparatif) | 0 % | 0 % | OK | OK |
| **F2** (comparatif) | 0 % | 3,5 % | déphasage après 10 jours | déphasage après 4 jours |
| **F3** (comparatif) | 5 % de MT 100 T | 0 % | décantation après 1 jour | - |
| **F4** (comparatif) | 5 % de TIOVEIL AQ | 0 % | décantation après 1 jour | - |
| **F5** (invention) | 5 % de MT 100 T | 3,5 % | OK | OK |
| **F6** (invention) | 5 % de MT 100 T | 2 % | OK | OK |
| **F7** (invention) | 5 % de TIOVEIL AQ | 2,5 % | OK | OK |

## Revendications

1. Compositions cosmétiques à usage topique, en particulier pour la photoprotection de la peau et/ou des cheveux, comprenant, dans un support cosmétiquement acceptable de type émulsion huile-dans-eau dans lequel la taille moyenne des globules qui constituent la phase huileuse est comprise entre 100 nm et 1000 nm (nanomètres), des nanopigments minéraux à base d'oxydes métalliques à titre d'agents photoprotecteurs, caractérisées par le fait qu'elles contiennent en outre au moins un silicate mixte de métaux alcalins et/ou alcalino-terreux.

2. Compositions selon la revendication 1, caractérisées en ce que ladite taille moyenne des globules huileux est comprise entre 100 et 500 nm.

3. Compositions selon l'une quelconque des revendications 1 ou 2, caractérisées en ce qu'au moins 90 % en nombres desdits globules huileux présentent une taille comprise entre 100 et 1000 nm, de préférence entre 100 et 500 nm.

4. Compositions selon l'une quelconque des revendications précédentes, caractérisées en ce que la taille moyenne des particules primaires constituant lesdits nanopigments est comprise entre 5 et 100 nm.

5. Compositions selon la revendication 4, caractérisées en ce que ladite taille moyenne est comprise entre 10 et 50 nm.

6. Compositions selon l'une quelconque des revendications précédentes, caractérisées en ce que lesdits nanopigments sont choisis parmi les oxydes de titane, de zinc, de fer, de zirconium, de cérium et leurs mélanges, enrobés ou non.

7. Compositions selon la revendication 6, caractérisées en ce que les nanopigments sont à base d'oxyde de titane, enrobé ou non enrobé.

8. Compositions selon la revendication 7, caractérisées en ce que l'oxyde de titane est sous forme rutile, anatase ou amorphe.

9. Compositions selon l'une quelconque des revendications précédentes, caractérisées en ce qu'elles contiennent en outre au moins un filtre solaire organique actif dans l'UV-A et/ou l'UV-B.

10. Compositions selon l'une quelconque des revendications précédentes, caractérisées en ce qu'elles contiennent en outre au moins un agent émulsionnant.

11. Compositions selon la revendication 10, caractérisées en ce que la teneur en agent(s) émulsionnant(s) est comprise entre 0,5 et 40 % en poids, de préférence entre 2 et 10 % en poids, par rapport au poids total de la composition.

12. Compositions selon l'une quelconque des revendications précédentes, caractérisées en ce qu'elles contiennent en outre au moins un adjuvant choisi parmi les épaississants ioniques ou non ioniques, les adoucissants, les antioxydants, les opacifiants, les stabilisants, les émollients, les agents répulsifs contre les insectes, les agents hydratants, les vitamines, les parfums, les conservateurs, les charges, les séquestrants et les colorants.

13. Compositions selon l'une quelconque des revendications précédentes, caractérisées en ce que la teneur en poids de la phase aqueuse représente de 50 à 95 %, de préférence de 70 à 90 %, du poids total de la composition.

14. Compositions selon l'une quelconque des revendications précédentes, caractérisées en ce que la teneur en poids de la phase huileuse représente de 5 à 50 %, de préférence de 10 à 30 %, du poids total de la composition.

15. Compositions selon l'une quelconque des revendications précédentes, caractérisées en ce que la teneur en poids des nanopigments représente de 0,5 à 40 %, de préférence de 1 à 30 %, du poids total de la composition.

16. Compositions selon l'une quelconque des revendications précédentes, caractérisées en ce que la teneur en poids des silicates mixtes représente de 0,05 à 5%, de préférence de 0,1 à 3,5 %, du poids total de la composition.

17. Compositions selon l'une quelconque des revendications précédentes, caractérisées en ce que le ou les métaux alcalins rentrant dans la composition du silicate mixte sont choisis parmi le lithium, le sodium et le potassium.

18. Compositions selon l'une quelconque des revendications précédentes, caractérisées en ce que le ou les métaux alcalino-terreux rentrant dans la composition du silicate mixte sont choisis parmi le magnésium et le calcium.

19. Compositions selon l'une quelconque des revendications précédentes, caractérisées en ce que lesdits silicates mixtes sont choisis parmi les silicates mixtes qui contiennent au moins un métal alcalino-terreux.

20. Compositions selon la revendication 19, caractérisées en ce que lesdits silicates mixtes contiennent en outre au moins un métal alcalin.

21. Compositions selon la revendication 20, caractérisées en ce que le silicate mixte est un silicate mixte de magnésium, de lithium et de sodium.

22. Procédé de préparation d'une composition telle que définie à l'une quelconque des revendications précédentes, caractérisé par le fait qu'il consiste à introduire dans une émulsion préalablement réalisée ou en cours de réalisation de type huile-dans-eau dans laquelle la taille moyenne des globules qui constituent la phase huileuse est comprise entre 100 nm et 1000 nm (nanomètres) et contenant des nanopigments minéraux à base d'oxydes métalliques à titre d'agents photoprotecteurs, un ou des silicates mixtes de métaux alcalins et/ou alcalino-terreux

23. Procédé de préparation selon la revendication 22, caractérisé par le fait qu'il comprend les étapes suivantes :
(i) on mélange, en présence d'un système émulsionnant convenable et sous agitation, une phase grasse d'une part et une phase aqueuse d'autre part, ledit mélange se faisant à une température supérieure à la température d'inversion de phase (TIP) du milieu, de manière à obtenir une émulsion de type eau-dans-huile,
(ii) on ramène la température de l'émulsion ainsi obtenue à une température inférieure à ladite température d'inversion de phase, ce par quoi l'on obtient une émulsion ultrafine de type huile-dans-eau,
(iii) on procède à une introduction de nanopigments minéraux et/ou de silicates mixtes lors de la mise en oeuvre de l'étape (i) et/ou à l'issue de l'étape (ii).

24. Procédé selon la revendication 23, caractérisé en ce que l'introduction des silicates se fait à l'issue de l'étape (ii).

25. Procédé selon la revendication 23 ou 24, caractérisé en ce que le ou les agents émulsionnants sont de type non ioniques et sont choisis, seuls ou en mélanges, parmi les alcools gras polyoxyéthylénes et/ou polyoxypropylénés, et les esters d'acides gras et de polyols, éventuellement polyoxyéthylénés et/ou polyoxypropylénés.

26. Procédé selon la revendication 25, caractérisé en ce que le système émulsionnant présente un HLB global compris entre 9,5 et 11,5 environ, et de préférence proche de 10.

27. Procédé de stabilisation d'une émulsion de type huile-dans-eau dans laquelle la taille moyenne des globules qui constituent la phase huileuse est comprise entre 100 nm et 1000 nm (nanomètres) et contenant des nanopigments minéraux à base d'oxydes métalliques à titre d'agents photoprotecteurs, caractérisé par le fait qu'il consiste à introduire dans ladite émulsion, pendant et/ou après la synthèse de cette dernière, un ou des silicates mixtes de métaux alcalins et/ou alcalino-terreux.

28. Utilisation d'au moins un silicate mixte de métaux alcalins et/ou alcalino-terreux pour la stabilisation d'une émulsion de type huile-dans-eau dans laquelle la taille moyenne des globules qui constituent la phase huileuse est comprise entre 100 nm et 1000 nm (nanomètres) et contenant des nanopigments minéraux à base d'oxydes métalliques à titre d'agents photoprotecteurs.

29. Utilisation des compositions définies à l'une quelconque des revendications 1 à 21 comme, ou pour la fabrication de, compositions cosmétiques pour la protection de la peau et/ou des cheveux contre le rayonnement ultraviolet, en particulier le rayonnement solaire.

30. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 21.

## Claims

1. Cosmetic compositions for topical use, in particular for photo-protection of the skin and/or of the hair, comprising, in a cosmetically acceptable vehicle of oil-in-water emulsion type in which the average size of the globules which constitute the oily phase is between 100 nm and 1000 nm (nanometres), inorganic nanopigments based on metal oxides as photo-protective agents, characterized in that they additionally contain at least one mixed silicate of alkali metals and/or alkaline-earth metals.

2. Compositions according to Claim 1, characterized in that the said average size of the oily globules is between 100 and 500 nm.

3. Compositions according to either of Claims 1 and 2, characterized in that at least 90 %, in numerical terms, of the said oily globules is between 100 and 1000 nm in size, and preferably between 100 and 500 nm.

4. Compositions according to any one of the preceding claims, characterized in that the average size of the primary particles constituting the said nanopigments is between 5 and 100 nm.

5. Compositions according to Claim 4, characterized in that the said average size is between 10 and 50 nm.

6. Compositions according to any one of the preceding claims, characterized in that the said nanopigments are chosen from titanium, zinc, iron, zirconium and cerium oxides and mixtures thereof, which may or may not be coated.

7. Compositions according to Claim 6, characterized in that the nanopigments are based on coated or uncoated titanium oxide.

8. Compositions according to Claim 7, characterized in that the titanium oxide is in rutile, anatase or amorphous form.

9. Compositions according to any one of the preceding claims, characterized in that they also contain at least one organic sunscreen agent which is active in UV-A and/or UV-B.

10. Compositions according to any one of the preceding claims, characterized in that they also contain at least one emulsifier.

11. Compositions according to Claim 10, characterized in that the content of emulsifier(s) is between 0.5 and 40 % by weight, and preferably between 2 and 10 % by weight, relative to the total weight of the composition.

12. Compositions according to any one of the preceding claims, characterized in that they also contain at least one adjuvant chosen from ionic or nonionic thickeners, demulcents, antioxidants, opacifiers, stabilizers, emollients, insect repellents, moisturizing agents, vitamins, perfumes, preservatives, fillers, sequestering agents and colorants.

13. Compositions according to any one of the preceding claims, characterized in that the content by weight of the aqueous phase represents from 50 to 95 %, and preferably from 70 to 90 %, of the total weight of the composition.

14. Compositions according to any one of the preceding claims, characterized in that the content by weight of the oily phase represents from 5 to 50 %, and preferably from 10 to 30 %, of the total weight of the composition.

15. Compositions according to any one of the preceding claims, characterized in that the content by weight of the nanopigments represents from 0.5 to 40 %, and preferably from 1 to 30 %, of the total weight of the composition.

16. Compositions according to any one of the preceding claims, characterized in that the content by weight of the mixed silicates represents from 0.05 to 5 %, and preferably from 0.1 to 3.5 %, of the total weight of the composition.

17. Compositions according to any one of the preceding claims, characterized in that the alkali metal or metals participating in the composition of the mixed silicate are chosen from lithium, sodium and potassium.

18. Compositions according to any one of the preceding claims, characterized in that the alkaline-earth metal or metals participating in the composition of the mixed silicate are chosen from magnesium and calcium.

19. Compositions according to any one of the preceding claims, characterized in that the said mixed silicates are chosen from mixed silicates which contain at least one alkaline-earth metal.

20. Compositions according to Claim 19, characterized in that the said mixed silicates also contain at least one alkali metal.

21. Compositions according to Claim 20, characterized in that the mixed silicate is a mixed silicate of magnesium, of lithium and of sodium.

22. Process for the preparation of a composition as defined in any one of the preceding claims, characterized in that it consists in introducing into an emulsion, which has been prepared beforehand or which is in the process of being prepared, of oil-in-water type in which the average size of the globules which constitute the oily phase is between 100 nm and 1000 nm (nanometres) and containing inorganic nanopigments based on metal oxides as photo-protective agents, one or more mixed silicates of alkali metals and/or alkaline-earth metals.

23. Preparation process according to Claim 22, characterized in that it comprises the following steps:
(i) a fatty phase, on the one hand, and an aqueous phase, on the other hand, are mixed together in the presence of a suitable emulsifying system and with stirring, the said mixing being performed at a temperature above the phase inversion temperature (PIT) of the medium, so as to obtain an emulsion of water-in-oil type,
(ii) the temperature of the emulsion thus obtained is brought to a temperature below the said phase inversion temperature, whereby an ultrafine emulsion of oil-in-water type is obtained,
(iii) inorganic nanopigments and/or mixed silicates are then introduced while step (i) is being carried out and/or after completion of step (ii).

24. Process according to Claim 23, characterized in that the silicates are introduced after completion of step (ii).

25. Process according to Claim 23 or 24, characterized in that the emulsifier or emulsifiers are of nonionic type and are chosen, alone or as mixtures, from polyoxyethylenated and/or polyoxypropylenated fatty alcohols and fatty acid esters of polyols, which are optionally polyoxyethylenated and/or polyoxypropylenated.

26. Process according to Claim 25, characterized in that the emulsifying system has an overall HLB between 9.5 and 11.5 approximately, and preferably close to 10.

27. Process for stabilizing an emulsion of oil-in-water type in which the average size of the globules which constitute the oily phase is between 100 nm and 1000 nm (nanometres) and containing inorganic nanopigments based on metal oxides as photo-protective agents, characterized in that it consists in introducing into the said emulsion, during and/or after synthesis of this emulsion, one or more mixed silicates of alkali metals and/or alkaline-earth metals.

28. Use of at least one mixed silicate of alkali metals and/or alkaline-earth metals for the stabilization of an emulsion of oil-in-water type in which the average size of the globules which constitute the oily phase is between 100 nm and 1000 nm (nanometres) and containing inorganic nanopigments based on metal oxides as photo-protective agents.

29. Use of the compositions defined in any one of Claims 1 to 21 as, or for the manufacture of, cosmetic compositions for the protection of the skin and/or the hair against ultraviolet radiation, in particular solar radiation.

30. Cosmetic treatment process for protecting the skin and/or the hair against ultraviolet radiation, in particular solar radiation, characterized in that it consists in applying to the latter an effective amount of a composition as defined in any one of Claims 1 to 21.

## Patentansprüche

1. Kosmetische Zusammensetzungen zur topischen Anwendung, insbesondere zum Lichtschutz der Haut und/oder der Haare, die in einem kosmetisch akzeptablen Trägerstoff vom Typ einer Öl-in-Wasser-Emulsion, in der die mittlere Teilchengröße der Kügelchen, die die Fettphase bilden, im Bereich von 100 bis 1000 nm liegt, anorganische Nanopigmente auf der Basis von Metalloxiden als Lichtschutzmittel enthalten,
**dadurch gekennzeichnet,** daß sie ferner mindestens ein gemischtes Silicat von Alkali- und/oder Erdalkalimetallen enthalten.

2. Zusammensetzungen nach Anspruch 1, dadurch gekennzeichnet, daß die mittlere Teilchengröße der Ölkügelchen im Bereich von 100 bis 500 nm liegt.

3. Zusammensetzungen nach Anspruch 1 oder 2, dadurch gekennzeichnet, daß zahlenmäßig mindestens 90 % der Ölkügelchen eine Teilchengröße im Bereich von 100 bis 1000 nm und vorzugsweise im Bereich von 100 bis 500 nm aufweisen.

4. Zusammensetzungen nach einem der vorhergehenden Ansprüche, dadurch gekennzeichnet, daß die mittlere Teilchengröße der die Nanopigmente bildenden Primärteilchen im Bereich von 5 bis 100 nm liegt.

5. Zusammensetzungen nach Anspruch 4, dadurch gekennzeichnet, daß die mittlere Teilchengröße im Bereich von 10 bis 50 nm liegt.

6. Zusammensetzungen nach einem der vorhergehenden Ansprüche, dadurch gekennzeichnet, daß die Nanopigmente unter Titanoxid, Zinkoxid, Eisenoxid, Zirkoniumoxid, Ceroxid und ihren Gemischen ausgewählt sind, wobei sie gegebenenfalls umhüllt sind.

7. Zusammensetzungen nach Anspruch 6, dadurch gekennzeichnet, daß die Nanopigmente auf der Basis von Titanoxid vorliegen und gegebenenfalls umhüllt sind.

8. Zusammensetzungen nach Anspruch 7, dadurch gekennzeichnet, daß das Titanoxid in Form von Rutil oder Anatas oder in amorpher Form vorliegt.

9. Zusammensetzungen nach einem der vorhergehenden Ansprüche, dadurch gekennzeichnet, daß sie ferner mindestens ein organisches Sonnenschutzfilter enthalten, das im UV-A-Bereich und/oder UV-B-Bereich wirksam ist.

10. Zusammensetzungen nach einem der vorhergehenden Ansprüche, dadurch gekennzeichnet, daß sie ferner mindestens einen Emulgator enthalten.

11. Zusammensetzungen nach Anspruch 10, dadurch gekennzeichnet, daß der Gehalt an Emulgator(en) im Bereich von 0,5 bis 40 Gew.-% und vorzugsweise von 2 bis 10 Gew.-%, bezogen auf das Gesamtgewicht der Zusammensetzung, liegt.

12. Zusammensetzungen nach einem der vorhergehenden Ansprüche, dadurch gekennzeichnet, daß sie ferner mindestens einen Zusatzstoff enthalten, der unter ionischen oder nichtionischen Verdickungsmitteln, Mitteln für die Geschmeidigkeit, Antioxidantien, Trübungsmitteln, Stabilisatoren, Mitteln für die Weichheit, insektenabwehrenden Mitteln, Hydratisierungsmitteln, Vitaminen, Parfums, Konservierungsmitteln, Füllstoffen, Maskierungsmitteln und Färbemitteln ausgewählt ist.

13. Zusammensetzungen nach einem der vorhergehenden Ansprüche, dadurch gekennzeichnet, daß der Gehalt an wäßriger Phase 50 bis 95 Gew.-% und vorzugsweise 70 bis 90 Gew.-% des Gesamtgewichts der Zusammensetzung beträgt.

14. Zusammensetzungen nach einem der vorhergehenden Ansprüche, dadurch gekennzeichnet, daß der Gehalt an Ölphase 5 bis 50 Gew.-% und vorzugsweise 10 bis 30 Gew.-% des Gesamtgewichts der Zusammensetzung beträgt.

15. Zusammensetzungen nach einem der vorhergehenden Ansprüche, dadurch gekennzeichnet, daß der Gehalt an Nanopigmenten 0,5 bis 40 Gew.-% und vorzugsweise 1 bis 30 Gew.-% des Gesamtgewichts der Zusammensetzung beträgt.

16. Zusammensetzungen nach einem der vorhergehenden Ansprüche, dadurch gekennzeichnet, daß der Gehalt an gemischten Silicaten 0,05 bis 5 Gew.-% und vorzugsweise 0,1 bis 3,5 Gew.-% des Gesamtgewichts der Zusammensetzung beträgt.

17. Zusammensetzungen nach einem der vorhergehenden Ansprüche, dadurch gekennzeichnet, daß das oder die in der Zusammensetzung des gemischten Silicats vorliegenden Alkalimetalle unter Lithium, Natrium und Kalium ausgewählt sind.

18. Zusammensetzungen nach einem der vorhergehenden Ansprüche, dadurch gekennzeichnet, daß das oder die in der Zusammensetzung des gemischten Silicats vorliegenden Erdalkalimetalle unter Magnesium und Calcium ausgewählt sind.

19. Zusammensetzungen nach einem der vorhergehenden Ansprüche, dadurch gekennzeichnet, daß die gemischten Silicate unter den gemischten Silicaten ausgewählt sind, die mindestens ein Erdalkalimetall enthalten.

20. Zusammensetzungen nach Anspruch 19, dadurch gekennzeichnet, daß die gemischten Silicate ferner mindestens ein Alkalimetall enthalten.

21. Zusammensetzungen nach Anspruch 20, dadurch gekennzeichnet, daß das gemischte Silicat ein gemischtes Silicat mit Magnesium, Lithium und Natrium ist.

22. Verfahren zur Herstellung einer Zusammensetzung nach einem der vorhergehenden Ansprüche, dadurch gekennzeichnet, daß es darin besteht, ein oder mehrere gemischte Silicate von Alkali- und/oder Erdalkalimetallen in eine vorher hergestellte Öl-in-Wasser-Emulsion oder bei ihrer Herstellung zuzusetzen, wobei in der Emulsion die mittlere Größe der Kügelchen, die die Fettphase bilden, im Bereich von 100 bis 1000 nm liegt und die Emulsion anorganische Nanopigmente auf der Basis von Metalloxiden als Lichtschutzmittel enthält.

23. Verfahren nach Anspruch 22, dadurch gekennzeichnet, daß es folgende Schritte umfaßt:
(i) Mischen einer Fettphase einerseits und einer wäßrigen Phase andererseits in Gegenwart eines Emulgatorsystems unter Rühren, wobei das Gemisch bei einer Temperatur über der Phaseninversionstemperatur (PIT) des Mediums gebildet wird, um eine Wasser-in-Öl-Emulsion zu erhalten,
(ii) Temperaturerniedrigung der so erhaltenen Emulsion auf eine Temperatur unter der Phaseninversionstemperatur, wodurch eine ultrafeine Öl-in-Wasser-Emulsion erhalten wird,
(iii) Zusatz von anorganischen Nanopigmenten und/oder gemischten Silicaten während der Durchführung von Schritt (i) und/oder am Ende von Schritt (ii).

24. Verfahren nach Anspruch 23, dadurch gekennzeichnet, daß der Zusatz der Silicate am Ende von Schritt (ii) durchgeführt wird.

25. Verfahren nach Anspruch 23 oder 24, dadurch gekennzeichnet, daß der oder die Emulgatoren vom nichtionischen Typ sind und allein oder als Gemisch unter polyethoxylierten und/oder polypropoxylierten Fettalkoholen und gegebenenfalls polyethoxylierten und/oder polypropoxylierten Estern von Fettsäuren mit Polyolen ausgewählt sind.

26. Verfahren nach Anspruch 25, dadurch gekennzeichnet, daß das Emulgatorsystem einen HLB-Gesamtwert im Bereich von etwa 9,5 bis 11,5 und vorzugsweise in der Nähe von 10 aufweist.

27. Verfahren zur Stabilisierung einer Öl-in-Wasser-Emulsion, bei der die mittlere Teilchengröße der Kügelchen, die die Ölphase bilden, im Bereich von 100 bis 1000 nm liegt und die anorganische Nanopigmente auf der Basis von Metalloxiden als Lichtschutzmittel enthalten, dadurch gekennzeichnet, daß es darin besteht, der Emulsion während und/oder nach ihrer Herstellung ein oder mehrere gemischte Silicate von Alkali- und/oder Erdalkalimetallen zuzusetzen.

28. Verwendung mindestens eines gemischten Silicats von Alkali- und/oder Erdalkalimetallen zur Stabilisierung einer Öl-in-Wasser-Emulsion, bei der die mittlere Teilchengröße der Kügelchen, die die Ölphase bilden, im Bereich von 100 bis 1000 nm liegt und die anorganische Nanopigmente auf der Basis von Metalloxiden als Lichtschutzmittel enthält.

29. Verwendung der Zusammensetzungen nach einem der Ansprüche 1 bis 21 als oder zur Herstellung von kosmetischen Zusammensetzungen zum Schutz der Haut und/oder der Haare gegen UV-Strahlung, insbesondere vor Sonnenstrahlung.

30. Verfahren zur kosmetischen Behandlung zum Schutz der Haut und/oder der Haare gegen UV-Strahlung, insbesondere Sonnenstrahlung, dadurch gekennzeichnet, daß es darin besteht, auf die Haut und/oder die Haare eine wirksame Menge einer Zusammensetzung nach einem der Ansprüche 1 bis 21 aufzutragen.
